(19) Europäisches Patentamt
European Patent Office
Office européen des brevets

(11) Numéro de publication: **0 017 294**
**B1**

(12) # FASCICULE DE BREVET EUROPEEN

(45) Date de publication du fascicule du brevet: **31.08.83**

(21) Numéro de dépôt: **80200289.9**

(22) Date de dépôt: **28.03.80**

(51) Int. Cl.³: **C 07 D 301/32,**
C 07 D 303/04,
C 07 D 303/08,
C 07 D 303/14

(54) **Procédé pour l'épuration d'oxydes d'oléfines.**

(30) Priorité: **06.04.79 FR 7909121**

(43) Date de publication de la demande:
**15.10.80 Bulletin 80/21**

(45) Mention de la délivrance du brevet:
**31.08.83 Bulletin 83/35**

(84) Etats contractants désignés:
**AT BE CH DE FR GB IT LU NL SE**

(56) Documents cités:
**FR - A - 2 317 296**
**GB - A - 2 002 376**

(73) Titulaire: **PROPYLOX (Société Anonyme)**
**12, avenue de la Renaissance**
**B-1040 Bruxelles (BE)**

(72) Inventeur: **Hardy, Nicolas**
**Rue de la Taille, 52**
**B-5790 Jemeppe-sur-Sambre (BE)**
Inventeur: **Durieux, Teddy**
**Avenue des Acacias, 46**
**B-5790 Jemeppe-sur-Sambre (BE)**

(74) Mandataire: **Meyers, Liliane et al,**
**Solvay & Cie Département de la propriété industrielle 310, rue de Ransbeek**
**B-1120 Bruxelles (BE)**

**EP 0 017 294 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

Procédé pour l'épuration d'oxydes d'oléfines

La présente invention concerne un procédé pour l'épuration d'oxydes d'oléfines. Elle concerne plus particulièrement l'épuration des oxydes d'oléfines obtenus par époxydation des oléfines correspondantes au moyen de composés peroxydés.

Lors de l'époxydation des oléfines au moyen de composés peroxydés tels que le peroxyde d'hydrogène ou les peracides carboxyliques, on obtient à la sortie du réacteur d'époxydation ou après une rectification préliminaire, des oxydes d'oléfines contenant diverses impuretés telles que l'eau, les glycols, les aldéhydes, les alcools, l'oléfine n'ayant pas réagi et le solvant éventuellement mis en oeuvre. Certains de ces produits, qui se révèlent gênant lors de l'utilisation des oxydes d'oléfines, par exemple comme monomère pour la fabrication de polymères, sont très difficiles à éliminer par les techniques classiques de distillation. En outre, au cours des nombreuses étapes de distillation nécessaires à leur élimination, on observe la formation de quantités importantes de sous-produits tels que des esters et des glycols.

Ainsi, lors de la fabrication d'oxyde de propylène par époxydation du propylène au moyen d'une solution organique de peracide (brevet belge 847 664 déposé le 27 octobre 1976 au nom de BAYER AG et DEGUSSA), il se forme du propylène glycol au cours des étapes de séparation du mélange réactionnel en ses divers constituants.

Pour éliminer les esters présents dans l'oxyde de propylène on a proposé (demande de brevet japonais 47/18812 déposée le 16 février 1971 au nom de MITSUBISHI CHEM. IND. LTD.) de traiter l'oxyde de propylène dans une colonne à distiller en présence d'une solution aqueuse d'un agent de saponification. Cette technique présente cependant l'inconvénient de provoquer de fortes corrosions, dans le bouilleur de la colonne à distiller.

On a également proposé dans la demande de brevet GB - A - 2002376 (Halcon Research and Development Corp), d'éliminer le méthanol et l'acétone présents dans de l'oxyde de propylène par distillation en présence d'eau. Cette technique, qui nécessite la séparation préalable du solvant de la réaction d'époxydation, présente également l'inconvénient de provoquer de fortes corrosions ainsi que la formation de produits secondaires dans le bouilleur de la colonne à distiller.

La présente invention a pour but de fournir un procédé pour l'épuration d'oxydes d'oléfines qui permet de pallier ces inconvénients et, notamment, d'épurer efficacement les oxydes d'oléfines tout en évitant la formation de produits secondaires et la corrosion.

Le procédé selon l'invention est en outre particulièrement aisé à réaliser. Il nécessite un nombre réduit de colonnes à distiller tout en permettant de séparer efficacement les aldéhydes.

L'invention concerne à cet effet un procédé pour l'épuration d'oxydes d'oléfines contenant de 2 à 20 atomes de carbone dans leur molécule à partir d'un mélange contenant l'oxyde d'oléfine et des glycols, des esters, des aldéhydes, des alcools ou des cétones comme impuretés par distillation en colonne du mélange en présence d'une petite quantité d'eau de manière à obtenir l'oxyde d'oléfine comme distillat, la quantité totale d'eau entrant dans la colonne étant de 0,05 à 20% du poids de l'alimentation en mélange contenant l'oxyde d'oléfine à épurer, selon lequel on distille un mélange contenant un solvant utilisé lors de la fabrication de l'oxyde d'oléfine de manière à recueillir le solvant au pied de la colonne, on prélève dans la colonne, sous le niveau d'introduction de l'eau, une fraction de liquide de ruissellement entre le point d'introduction du mélange et le dixième inférieur de la colonne, on soumet la fraction prélevée à une décantation de manière à séparer une phase aqueuse que l'on rejette et une phase organique, et on renvoie la phase organique dans la colonne en un point situé sous le point de prélèvement.

La quantité relative du liquide de ruissellement prélevée n'est pas critique en elle-même. Généralement, elle est adaptée en fonction de la quantité d'eau présente dans l'oxyde de propylène. En général, on s'arrange pour prélever au moins 10% du liquide de ruissellement.

Le prélèvement du liquide de ruissellement se fait en un point situé sous le point d'introduction du mélange contenant l'oxyde d'oléfine à épurer. Il est choisi de préférence de manière à éviter une démixion de phases dans la colonne à distiller en dehors de l'endroit du prélèvement. De bons résultats ont été obtenus lorsque le point de prélèvement est situé entre le point d'introduction du mélange contenant l'oxyde d'oléfine à épurer et le dixième inférieur de la colonne.

Le prélèvement peut se faire selon diverses techniques. Il est recommandé de veiller à ce que le prélèvement ne gêne pas la montée des vapeurs chaudes dans la colonne. Une façon de faire qui a donné de bons résultats consiste à disposer dans la colonne un plateau ayant la forme d'un entonnoir très évasé, laissant passer latéralement les vapeurs chaudes, collectant le liquide de ruissellement et communiquant par un conduit avec un décanteur-séparateur qui peut quant à lui être situé dans ou hors de la colonne à distiller. Divers types de décanteurs-séparateurs connus en eux-mêmes peuvent être utilisés à cette fin.

Le renvoi à la colonne à distiller de la phase organique séparée après décantation de la fraction prélevée peut se faire en n'importe quel

point de la colonne dans le voisinage du, ou sous le point de prélèvement. De bons résultats ont été obtenus lorsque la phase organique est renvoyée dans la zone située directement sous le point de prélèvement.

Le procédé selon l'invention peut être réalisé en continu ou en semi-continu. De bons résultats ont été obtenus en collectant en continu une fraction du liquide de ruissellement en le soumettant en continue à la décantation et à la séparation, et en renvoyant en continu la phase organique à la colonne à distiller.

L'eau présente dans la colonne à distiller peut avoir diverses origines. Il peut s'agir de l'eau présente comme impureté dans l'oxyde d'oléfine. Elle peut également être ajoutée volontairement dans la colonne à distiller. Elle peut enfin également provenir simultanément de ces deux sources. Cette dernière solution s'est révélée avantageuse.

La quantité totale d'eau entrant dans la colonne à distiller n'est pas supérieure à 20% en poids du poids du mélange à distiller entrant dans la colonne. Des proportions en eau supérieures ne sont pas favorables car elles provoquent des réactions secondaires d'hydrolyse. De bons résultats ont été obtenus lorsque le rapport pondéral eau sur mélange à distiller alimentant la colonne ne dépasse pas 10%. Par ailleurs, pour arriver à extraire suffisamment certaines impuretés, on introduit dans la colonne une quantité totale d'eau égale à au moins 0,05% en poids du mélange alimentant la colonne. De bons résultats ont été obtenus lorsque cette quantité est égale à au moins 0,1% de celle de mélange.

L'eau introduite volontairement dans la colonne à distiller peut être de l'eau pure ou une solution aqueuse. En général, on préfère introduire dans la colonne une solution aqueuse dont le pH est supérieur à 7. Ces solutions peuvent contenir divers types de composés capables de conférer à l'eau un caractère basique. La nature de ces composés est quelconque. En général, ils sont choisis parmi les hydroxydes, les carbonates, les bicarbonates de métaux alcalins ou alcalino-terreux et leurs mélanges. De bons résultats ont été obtenus avec les hydroxydes alcalins et plus particulièrement l'hydroxyde de sodium.

Ces solutions aqueuses peuvent contenir des quantités variables de composés basiques. En général pour éviter la précipitation de certains sels dans la phase aqueuse soumise à la décantation on préfère utiliser des solutions aqueuses ne contenant pas plus de 20% en poids de ces composés basiques. De bons résultats ont été obtenus lorsque leurs teneurs ne dépassent pas 12% du poids de la solution. Par ailleurs on préfère en général utiliser des solutions contenant au moins 0,1% de leur poids de composés basiques. De bons résultats ont été obtenus quand ces solutions contiennent au moins 1% de leur poids de composés basiques.

L'eau ou les solutions aqueuses introduites volontairement dans la colonne sont introduites au-dessus du point de prélèvement du liquide de ruissellement. En général, elles sont introduites dans la colonne à un niveau identique ou plus élevé que celui de l'introduction du mélange contenant l'oxyde d'oléfine à épurer.

L'introduction de solutions aqueuses peut se faire en un ou plusieurs points; la solution aqueuse peut également être ajoutée à l'alimentation de la colonne.

Les conditions de fonctionnement de la colonne à distiller dans le procédé selon l'invention sont choisies essentiellement en fonction de la nature de l'oxyde d'oléfine à épurer. On peut, de ce fait, utiliser des températures et des pressions très variables.

La température est en général comprise entre 270 et 450 K. Des températures supérieures sont moins intéressantes car elles entraînent un risque de réactions secondaires. La pression est réglée en fonction de la température de manière à maintenir l'ébullition. Elle est le plus souvent comprise entre 0,001 MPa et 5 MPa.

Le procédé selon l'invention peut être utilisé pour épurer divers types d'oxydes d'oléfines comportant au moins un pont époxyde. Le procédé est appliqué de préférence aux oxydes d'oléfines qui contiennent des groupes

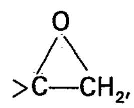 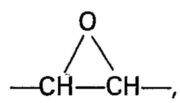 ou 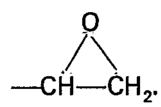

Les oxydes d'alkènes ou de cycloalkènes auxquels le procédé est applicable peuvent être non substitués ou substitués par un ou plusieurs substituants choisis parmi les groupes alkyles contenant en général de 1 à 4 atomes de carbone, les groupes cycloalkyles, les groupes aryles ou les substituants contenant des hétéroatomes. Parmi les oxydes d'alkènes ou de cycloalkènes non substitués ou substitués uniquement par des groupes alkyles, cycloalkyles, ou aryles auxquels le procédé suivant l'invention est applicable on peut citer plus particulièrement l'oxyde d'éthylène, l'oxyde de propylène, l'oxyde de méthylpropène, les oxydes de butènes, le dioxyde de butadiène, les oxydes de pentènes, les dioxydes de pentadiènes et notamment l'oxyde d'isoprène, les oxydes d'hexènes, les dioxydes d'hexadiènes, l'oxyde de diisobutylène, les oxydes d'octènes, les oxydes de nonènes, les oxydes de décènes, l'oxyde d'$\alpha$-pinène, l'oxyde de p.menthène, les oxydes de tri- et tétra-propylènes, l'oxyde de tétradécène, l'oxyde d'hexadécène, l'oxyde d'octadécène, l'oxyde de dodécène, l'oxyde d'eicosène, l'oxyde de styrène, l'oxyde de méthylstyrène, l'oxyde de vinyltoluène, les oxydes de vinylcyclo -hexane et -hexène, l'oxyde de cyclohexène, l'oxyde de limonène; le dioxyde de divinylbenzène et les oxydes de stilbènes.

Les oxydes d'alkènes et de cycloalkènes utilisables dans le procédé de l'invention peuvent également être substitués par un ou plusieurs substituants contenant des hétéroatomes tels que des atomes d'halogènes et plus particulièrement des atomes de chlore, de fluor et de brome, des groupes sulfoniques ou phosphoriques, des groupes hydroxy, alkoxy, carboxy, acyl, alkoxycarbonyl, aryloxycarbonyl, acyloxy, acylamino, arylamido, alkylamido, imido ou nitrilo.

Comme oxydes d'oléfines substitués utilisables on peut citer plus particulièrement l'épichlorhydrine, l'épibromhydrine, la méthylépichlorhydrine, la méthylépibromhydrine, le glycidol, le méthylglycidol, le méthoxyglycidol, l'éthoxyglycidol, les oxydes des esters d'acide acrylique, méthacrylique, maléique avec des alcools saturés ou insaturés, ainsi que les oxydes des esters d'acides carboxyliques saturés avec des alcools insaturés tels que l'alcool allylique et méthallylique.

Le procédé selon l'invention s'applique particulièrement bien à l'épuration d'oxyde de propylène, d'oxydes de butène, d'oxyde de cyclohexène, d'épichlorhydrine, d'oxyde de styrène, de diépoxybutane, et de glycidol. De très bons résultats ont été obtenus lors de l'épuration de mélanges à base d'oxyde de propylène.

Les mélanges contenant l'oxyde d'oléfine soumis à l'épuration, contiennent outre l'oxyde d'oléfine, des impuretés et divers types de produits selon le mode de fabrication mis en oeuvre pour fabriquer l'oxyde d'oléfine. Ces produits sont en général des solvants et des sous-produits de l'agent d'époxydation ainsi que des réactifs non transformés.

Les solvants présents dans les mélanges contenant l'oxyde d'oléfine peuvent être de natures très diverses selon le mode de fabrication de l'oxyde d'oléfine. Ils font en général partie des alcools, des esters carboxyliques, des éthers, des hydrocarbures halogénés, des hydrocarbures non substitués, des hydrocarbures substitués par des groupes nitro, les esters non acides d'acides nitrique, carbonique et phosphorique, les nitriles, les amides et leurs mélanges. Ces solvants sont présents en quantités variables dans le mélange contenant l'oxyde d'oléfine à épurer. En général le mélange à épurer peut contenir jusqu'à 90% en poids de solvant.

Le mélange contenant l'oxyde d'oléfine peut également contenir de l'oléfine non transformée et des résidus de l'agent d'époxydation tels que l'eau et les acides carboxyliques par exemple, ainsi que divers types de sous-produits parmi lesquels figurent des glycols, des esters, des aldéhydes, des alcools et des cétones. En général, les mélanges à épurer contiennent de 0,01 à 10% en poids d'eau et de 0,001 à 5% en poids de sous-produits. Si l'oxyde d'oléfine est obtenu selon un procédé au peracide, ils peuvent en outre contenir jusqu'à 25% en poids de l'acide carboxylique correspondant.

Si le mélange contenant l'oxyde d'oléfine n'a pas été soumis à une séparation préalable de l'oléfine non transformée au cours de la réaction d'époxydation, il peut contenir des quantités importantes d'oléfine pouvant atteindre et même dépasser 10 fois la quantité d'oxyde de propylène présente.

L'oxyde d'oléfine recueilli en tête de la colonne dans le procédé selon l'invention peut subir une distillation subséquente afin d'éliminer les produits légers tels que par exemple l'oléfine non transformée et les aldéhydes légers qui pourraient éventuellement être encore présents. Dans ce cas, on envoie avantageusement l'oxyde d'oléfine dans une colonne à distiller subséquente dont on soutire en tête les produits légers, dont l'oléfine non transformée, que l'on peut éventuellement récupérer. Dans la partie inférieure de cette colonne, on recueille l'oxyde d'oléfine parfaitement épuré de préférence en phase gazeuse et le liquide recueilli en pied de cette colonne est renvoyé dans la colonne fonctionnant selon l'invention.

Les impuretés éliminées essentiellement par le procédé selon l'invention sont l'eau, les esters, les aldéhydes et les glycols ainsi que les acides carboxyliques éventuellement sous forme de sels lorsque la solution aqueuse utilisée à l'épuration contient des composés basiques.

Le procédé selon l'invention permet d'obtenir des oxydes d'oléfines épurés qui conviennent bien comme monomère de polymérisation. Il peut être appliqué de façon particulièrement avantageuse à l'épuration d'oxydes d'oléfines obtenus par le procédé aux peracides.

Le procédé selon l'invention peut être réalisé dans des appareils tels que ceux représentés schématiquement aux figures 1 et 2 des dessins en annexe qui se rapportent à des modes de réalisation pratiques particuliers.

Selon le procédé schématisé à la figure 1, on introduit dans la colonne à distiller 30 par la voie 24 un mélange contenant l'oxyde d'oléfine, de petites quantités d'oléfine non transformée, un solvant et des impuretés.

Dans la colonne 30 est disposé un plateau 31 qui permet de collecter une partie du liquide de ruissellement de la colonne qui est envoyé par la voie 32 dans un décanteur 33 dans lequel on sépare une phase aqueuse que l'on soutire par la voie 34 d'une phase organique que l'on soutire par la voie 35 et qu'on recycle à l'aide de la pompe 36 par la voie 37 à la colonne 30.

Le liquide recueilli au pied de la colonne 30 par la voie 38 est partiellement envoyé par la voie 39 au bouilleur 41 d'où il est renvoyé par la voie 42 à la colonne 30. L'autre partie est recueillie par la voie 40. Ce liquide comprend principalement le solvant.

La solution aqueuse est introduite dans la colonne 30 par la voie 48.

Les vapeurs qui contiennent l'oxyde d'oléfine

quittent la colonne 30 par la voie 43, sont condensées en 44 et une partie du liquide issu du condenseur 44 par la voie 45 est renvoyée à la colonne par la voie 46 où elle assure le reflux et l'autre partie est envoyée par la voie 47 dans la colonne 49 pour en éliminer les légers.

L'oxyde d'oléfine épuré est recueilli par la voie 55 dans un des plateaux de la colonne, condensé en 56 et récupéré en 62.

Les vapeurs recueillies en 50 et qui contiennent notamment l'oléfine sont partiellement récupérées en 51 et partiellement envoyées par la voie 52 dans un condenseur 53 pour être recyclées par la voie 54 à la colonne 49 où elles assurent le reflux.

Le liquide recueilli au pied de la colonne 49 par la voie 57 est partiellement envoyé par la voie 59 au bouilleur 60 d'où il est renvoyé à la colonne 49 par la voie 61. L'autre partie du liquide recueilli au pied de la colonne 49 est envoyée par la voie 58 à la colonne 30.

A la figure 2, est schématisé un mode d'application de l'invention au traitement du produit d'époxydation des oléfines à l'intervention de peracides.

On introduit dans le réacteur 1 d'époxydation par la voie 3 l'oléfine et par la voie 2 une solution de peracide carboxylique. Le mélange contenant l'oxyde d'oléfine, l'acide carboxylique, le solvant et l'oléfine non transformée est envoyé par la voie 4 dans une colonne de stripping 5 d'où on soutire en tête par la voie 6 la plus grande partie de l'oléfine non transformée. Après condensation partielle préalable en 7, le liquide condensé est renvoyé par la voie 8 comme reflux à la colonne. Les vapeurs recueillies par la voie 10 sont envoyées dans la colonne d'absorption 11 où elles sont absorbées dans la solution de peracide carboxylique introduite par la voie 12. Les légers éventuels (gaz inertes) quittent la colonne d'absorption par la voie 13.

Une variante, non représentée à la figure 2, consiste à absorber dans tout ou partie du solvant recueilli par la voie 40, les vapeurs recueillies par la voie 10 ainsi qu'éventuellement l'oléfine fraîche et à envoyer la solution ainsi obtenue dans le réacteur d'époxydation 1.

Au pied de la colonne de stripping 5 on recueille par la voie 14 un liquide dont une partie est envoyée par la voie 15 dans le bouilleur de colonne 16 avant d'être renvoyée par la voie 17 à la colonne à distiller. L'autre partie du liquide recueilli par la voie 14 est envoyée par la voie 18 à la colonne à distiller 19. En pied de la colonne à distiller 19 on recueille par la voie 25 un liquide dont une partie est envoyée par la voie 27 au bouilleur de colonne 28 et ensuite par la voie 29 à la colonne 19. Le solde est recueilli par la voie 26; il comprend l'acide carboxylique et une partie du solvant de réaction.

Les vapeurs recueillies en tête de la colonne 19 par la voie 20 sont condensées en 21. Le condensat recueilli en 22 est renvoyé partiellement par la voie 23 à la colonne 19 où il assure le reflux et partiellement à la colonne 30 par la voie 24.

Les colonnes 30 et 49 fonctionnent de la même manière que dans la figure 1.

Les légers issus des condenseurs 44 et 53 sont récupérés respectivement par les voies 63 et 64 envoyés par la voie 10 à l'absorbeur 11.

Afin d'illustrer l'invention, sans pour autant en limiter la portée, on fournit ci-après un exemple pratique de réalisation, ainsi qu'un exemple donné à titre de comparaison.

## Exemple 1

Cet exemple concerne l'épuration d'un mélange contenant de l'oxyde de propylène obtenu par réaction de propylène avec de l'acide perpropionique en milieu 1,2-dichloréthane.

L'appareil est semblable à celui schématisé à la figure 1.

Dans la colonne 30 la pression est de 0,11 MPa et dans la colonne 49 elle est de 0,13 MPa. La colonne 30 est alimentée en continu par la voie 48 par une solution aqueuse contenant 5% d'hydroxyde de sodium à raison de 1,7 kg/heure.

La composition des flux de matière dans les différentes parties des deux zones de distillation est donnée au Tableau 1 ci-après. L'alimentation en produit à distiller est d'environ 100 kg/h. En tête de la colonne 30 on recueille 33 kg/h. Le recyclage de la colonne 49 vers la colonne 30 se fait avec un débit de 1 l/h. Le débit dans le flux 37 est de 200 kg/h.

TABLEAU 1

| | | voie 24 | voie 38 | voie 43 | voie 34 | voie 62 |
|---|---|---|---|---|---|---|
| propylène, | g/kg | 0,08 | — | 2,53 | | — |
| chlorure d'éthyle, | g/kg | 0,36 | — | 3,15 | | — |
| chlorure de vinyle, | g/kg | 0,02 | 0,01 | 0,5 | | — |
| dichloréthane, | g/kg | solde | solde | — | 6,6 | — |
| eau, | g/kg | 11 | 0,33 | 0,04 | solde | 0,03 |
| acétaldéhyde, | g/kg | 0,85 | 0,26 | 1,5 | 0,8 | 0,014 |
| propionaldéhyde, | g/kg | 0,35 | 0,00 | 0,00 | 4,8 | — |
| oxyde de propylène, | g/kg | 292 | 0,02 | solde | | solde |
| méthanol, | g/kg | 0,54 | 0,43 | 0,00 | 0,7 | — |
| éthanol, | g/kg | 0,60 | 0,05 | 0,00 | 17 | — |
| acide acétique, | g/kg | 0,40 | 0,00 | 0,06 | 13* | } 0,015 |
| acide propionique, | g/kg | 0,98 | 0,00 | 0,00 | 0.2* | |
| esters de glycol, | g/kg | 0,06 | 0,02 | 0,00 | | — |
| propylèneglycol et dimères, | g/kg | 0,04 | 0,36 | <0,01 | 4,3 | — |
| divers, | g/kg | 0,12 | 0,38 | 0,20 | 1,1 | — |

* sous forme de leurs sels de sodium

Après plusieurs jours de fonctionnement on n'observe pas de corrosion dans le bouilleur de la colonne 30.

Exemple 2 (de comparaison)

Le même essai a été réalisé que celui de l'exemple 1 mais en l'absence du dispositif permettant de récolter le liquide de ruissellement (entonnoir 31).

On observe alors des dépôts de solides (formiate, acétate, etc. de soude) qui tendent à boucher le thermosiphon en pied de colonne.

La teneur en propylèneglycol est dans ce cas très élevée: par la voie 38 on recueille un liquide contenant de 3 à 6,8 g/kg de propylèneglycol.

**Revendications**

1. Procédé pour l'épuration d'oxydes d'oléfines contenant de 2 à 20 atomes de carbone dans leur molécule à partir d'un mélange contenant l'oxyde d'oléfine et des glycols, des esters, des aldéhydes, des alcools ou des cétones comme impuretés par distillation en colonne du mélange, en présence d'une petite quantité d'eau, de manière à obtenir l'oxyde d'oléfine comme distillat, la quantité totale d'eau entrant dans la colonne étant de 0,05 à 20% du poids de l'alimentation en mélange contenant l'oxyde d'oléfine à épurer, caractérisé en ce qu'on distille un mélange contenant un solvant utilisé lors de la fabrication de l'oxyde d'oléfine de manière à recueillir le solvant au pied de la colonne, on prélève dans la colonne, sous le niveau d'introduction de l'eau, une fraction du liquide de ruissellement entre le point d'introduction du mélange et le dixième inférieur de la colonne, on soumet la fraction prélevée à une décantation de manière à séparer une phase aqueuse que l'on rejette et une phase organique, et on renvoie la phase organique dans la colonne en un point situé sous le point de prélèvement.

2. Procédé selon la revendication 1 caractérisé en ce que l'on introduit dans la colonne à distiller une solution aqueuse d'un composé capable de conférer un caractère basique à l'eau.

3. Procédé selon la revendication 2 caractérisé en ce que l'on introduit une solution aqueuse contenant de 0,1 à 20% en poids d'un composé basique.

4. Procédé selon la revendication 2 ou 3 caractérisé en ce que l'on met en oeuvre un

composé basique choisi parmi les hydroxydes, carbonates et bicarbonates de métaux alcalins et alcalino-terreux.

5. Procédé selon la revendication 4 caractérisé en ce que le composé basique est l'hydroxyde de sodium.

6. Procédé selon l'une quelconque des revendications 1 à 5 caractérisé en ce qu'il est appliqué à un mélange contenant jusqu'à 90% en poids de solvant.

7. Procédé selon l'une quelconque des revendications 1 à 6 caractérisé en ce qu'il est appliqué à un mélange contenant en outre de l'oléfine non transformée et des résidus de l'agent d'époxydation.

8. Procédé selon la revendication 7 caractérisé en ce qu'il est appliqué à un mélange obtenu par époxydation de l'oléfine correspondant à l'oxyde d'oléfine au moyen d'un peracide carboxylique et contenant de l'eau et de l'acide carboxylique dérivé du peracide.

9. Procédé selon la revendication 8 caractérisé en ce qu'il est appliqué à un mélange contenant jusqu'à 25% en poids d'acide carboxylique.

10. Procédé selon l'une quelconque des revendications 1 à 9 caractérisé en ce qu'il est appliqué à un mélange contenant de 0,01 à 10% en poids d'eau et de 0,001 à 5% en poids de glycols, esters, aldéhydes, alcools et cétones sous-produits de la fabrication de l'oxyde d'oléfine.

11. Procédé selon l'une quelconque des revendications 1 à 10 caractérisé en ce que l'on soumet l'oxyde d'oléfine recueilli comme distillat à une distillation subséquence dans une seconde colonne dont on soutire en tête les produits légers, on recueille en pied une fraction lourde que l'on recycle à la distillation du mélange contenant l'oxyde d'oléfine et on prélève dans la partie inférieure l'oxyde d'oléfine épuré.

12. Procédé selon l'une quelconque des revendications 1 à 11 caractérisé en ce qu'il est appliqué à l'épuration de mélanges contenant de l'oxyde d'oléfine choisi parmi l'oxyde de propylène, les oxydes de butène, l'oxyde de cyclohexène, l'épichlorhydrine, l'oxyde de styrène, le diépoxybutane et le glycidol.

13. Procédé selon la revendication 12 caractérisé en ce qu'il est appliqué à l'épuration de mélanges contenant l'oxyde de propylène.

**Patentansprüche**

1. Verfahren zur Reinigung von Olefin-Oxyden enthaltend 2 bis 20 Kohlenstoffatome in ihrem Molekül ausgehend von einer Mischung enthaltend das Olefin-Oxyd und Glykole, Ester, Aldehyde, Alkohole oder Ketone als Verunreinigungen durch Kolonnendestillation der Mischung in Anwesenheit einer geringen Wassermenge in der Weise, daß das Olefinoxyd als Destillat erhalten wird, wobei die gesamte Wassermenge, die in die Kolonne eintritt, 0,05 bis 20 Gew.-% bezogen auf die zugeführte, das zu reinigende Olefinoxyd enthaltende Mischung beträgt, dadurch gekennzeichnet, daß man eine Mischung destilliert die ein Lösungsmittel enthält, welches während der Herstellung des Olefinoxyds benutzt worden ist, in der Weise, daß man das Lösungsmittel am Fuß der Kolonne sammelt, man aus der Kolonne unterhalb des Niveaus der Wassereinführung eine Rieselfraktion der Flüssigkeit zwischen dem Punkt der Einführung der Mischung und dem unteren Zehntel der Kolonne abzieht, man die abgezogene Fraktion einer Dekantierung unterwirft in der Weise, daß eine wässrige Phase abgetrennt wird, die man verwirft, und eine organische Phase und man die organische Phase in die Kolonne zurückführt in einem Punkt, der unterhalb des Punktes des Abziehens liegt.

2. Verfahren nach Anspruch 1, dadurch gekennzeichnet, daß man in die Destillationskolonne eine wässrige Lösung einer Verbindung einführt, die geeignet ist, dem Wasser einen basischen Charakter zu verleihen.

3. Verfahren nach Anspruch 2, dadurch gekennzeichnet, daß man eine wässrige Lösung einführt, die 0,1 bis 20 Gew.-% einer basischen Verbindung enthält.

4. Verfahren nach Anspruch 2 oder 3, dadurch gekennzeichnet, daß man eine basische Verbindung einsetzt ausgewählt unter den Hydroxyden, Carbonaten und Bicarbonaten der Alkalimetalle und Erdalkalimetalle.

5. Verfahren nach Anspruch 4 dadurch gekennzeichnet, daß die basische Verbindung Natriumhydroxyd ist.

6. Verfahren nach einem der Ansprüche 1 bis 5, dadurch gekennzeichnet, daß es angewandt wird auf eine Mischung die bis zu 90 Gew.-% Lösungsmittel enthält.

7. Verfahren nach einem der Ansprüche 1 bis 6, dadurch gekennzeichnet, daß es angewandt wird auf eine Mischung die außerdem nicht umgewandeltes Olefin und restliches Epoxidierungsmittel enthält.

8. Verfahren nach Anspruch 7, dadurch gekennzeichnet, daß es angewandt wird auf eine Mischung erhalten durch Epoxidierung von Olefin entsprechend dem Olefinoxyd mittels einer Percarbonsäure, und enthaltend Wasser und Carbonsäure erzeugt aus der Percarbonsäure.

9. Verfahren nach Anspruch 8, dadurch gekennzeichnet, daß es angewandt wird auf eine Mischung enthaltend bis zu 25 Gew.-% Carbonsäure.

10. Verfahren nach einem der Ansprüche 1 bis 9, dadurch gekennzeichnet, daß es angewandt wird auf eine Mischung enthaltend 0,01 bis 10 Gew.-% Wasser und 0,01 bis 5 Gew.-% Glykole, Ester, Aldehyde, Alkohole und Ketone, Nebenprodukte der Herstellung des Olefinoxyds.

11. Verfahren nach einem der Ansprüche 1 bis 10 dadurch gekennzeichnet, daß man das als Destillat gesammelte Olefinoxyd einer nachfolgenden Destillation in einer zweiten Kolonne

unterwirft, aus der man am Kopf die leichten Produkte abzieht und am Fuß eine schwere Fraktion sammelt, die man zurückführt zu der Destillationsmischung enthaltend Olefinoxyd und man das gereinigte Olefinoxyd in dem unteren Teil abzieht.

12. Verfahren nach einem der Ansprüche 1 bis 11, dadurch gekennzeichnet, daß es angewandt wird auf die Reinigung von Mischungen enthaltend Olefinoxyd ausgewählt unter Propylenoxyd, den Butenoxyden, Cyclohexenoxyd, Epichlorhydrin, Styroloxyd, Diepoxybutan und Glycidol.

13. Verfahren nach Anspruch 12, dadurch gekennzeichnet, daß es angewandt wird auf die Reinigung von Mischungen enthaltend Propylenoxyd.

**Claims**

1. Process for the purification of olefine oxides containing from 2 to 20 carbon atoms in their molecule, from a mixture containing the olefine oxide and glycols, esters, aldehydes, alcohols or ketones as impurities, by subjecting the mixture to column distillation, in the presence of a small amount of water, so as to obtain the olefine oxide as the distillate, the total amount of water entering the column being 0.05 to 20% of the weight of the feed mixture containing the olefine oxide to be purified, characterised in that a mixture containing a solvent used during the manufacture of the olefine oxide is distilled so as to collect the solvent at the bottom of the column, a fraction of the down-flow liquid is withdrawn from the column, below the level of introduction of the water, between the point of introduction of the mixture and the lower tenth of the column, the withdrawn fraction is subjected to decantation so as to separate off an aqueous phase, which is discarded, and an organic phase, and the organic phase is returned to the column at a point located below the withdrawal point.

2. Process according to Claim 1, characterised in that an aqueous solution of a compound capable of imparting a basic character to the water is introduced into the distillation column.

3. Process according to Claim 2, characterised in that an aqueous solution containing from 0.1 to 20% by weight of a basic compound is introduced.

4. Process according to Claim 2 or 3, characterised in that the basic compound used is chosen from amongst alkali metal and alkaline earth metal hydroxides, carbonates and bicarbonates.

5. Process according to Claim 4, characterised in that the basic compound is sodium hydroxide.

6. Process according to any one of Claims 1 to 5, characterised in that it is applied to a mixture containing up to 90% by weight of solvent.

7. Process according to any one of Claims 1 to 6, characterised in that it is applied to a mixture additionally containing unconverted olefine and residues from the epoxidising agent.

8. Process according to Claim 7, characterised in that it is applied to a mixture obtained by epoxidation of the olefin corresponding to the olefine oxide, by means of a percarboxylic acid, and containing water and the carboxylic acid derived from the peracid.

9. Process according to Claim 8, characterised in that it is applied to a mixture containing up to 25% by weight of carboxylic acid.

10. Process according to any one of Claims 1 to 9, characterised in that it is applied to a mixture containing from 0.01 to 10% by weight of water and from 0.001 to 5% by weight of glycols, esters, aldehydes, alcohols and ketones, which are by-products from the manufacture of the olefine oxide.

11. Process according to any one of Claims 1 to 10, characterised in that the olefine oxide collected as the distillate is subjected to a subsequent distillation in a second column, from which the light products are withdrawn at the top, a heavy fraction is collected at the bottom and recycled into the distillation of the mixture containing the olefine oxide, and the purified olefine oxide is withdrawn from the lower part.

12. Process according to any one of Claims 1 to 11, characterised in that it is applied to the purification of mixtures containing an olefine oxide chosen from amongst propylene oxide, butene oxides, cyclohexene oxide, epichlorohydrin, styrene oxide, diepoxybutane and glycidol.

13. Process according to Claim 12, characterised in that it is applied to the purification of mixtures containing propylene oxide.

FIG 1

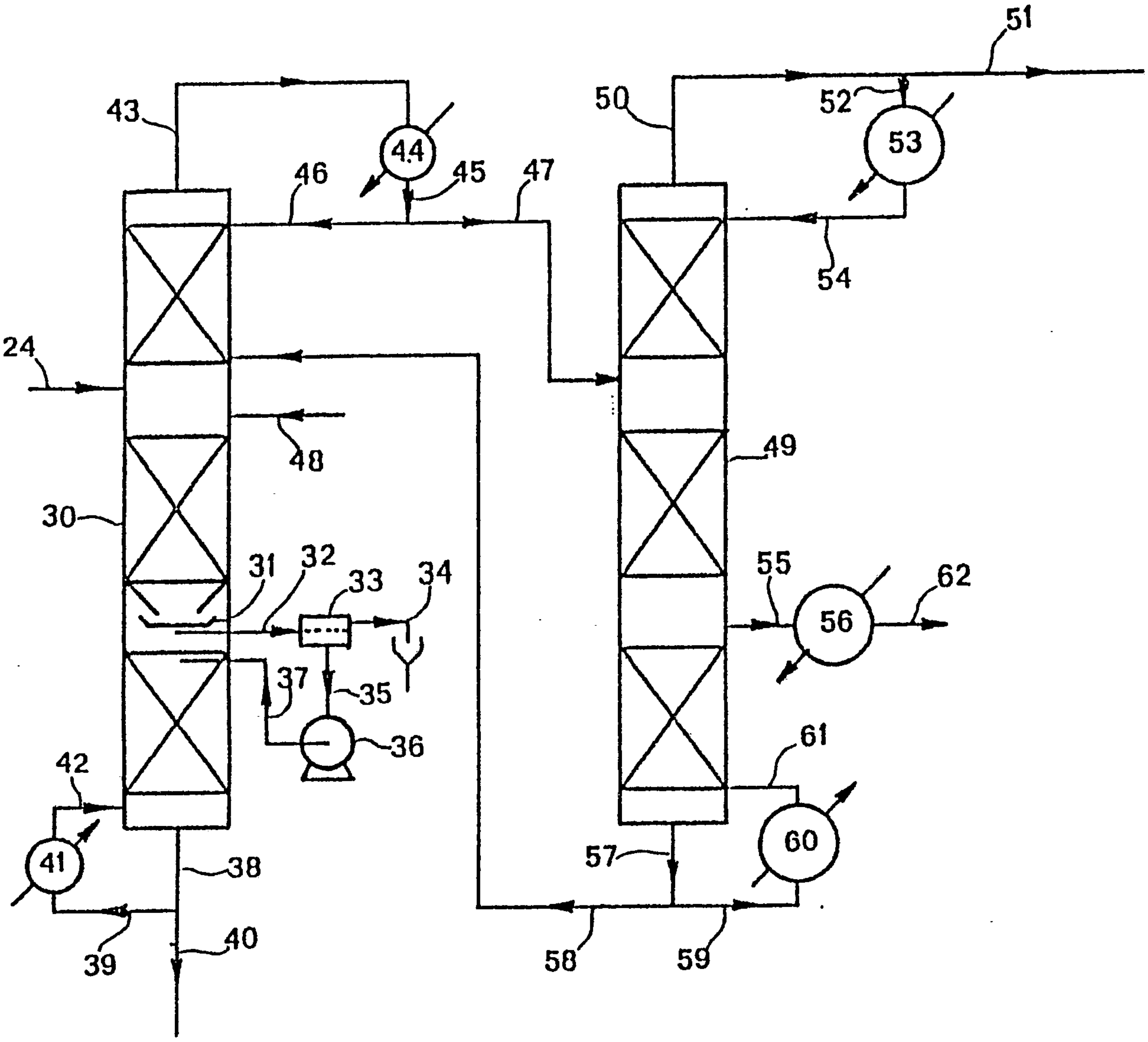
51
43
50
52
44
53
46
45
47
54
24
48
49
30
31
32
33
34
55
56
62
37
35
36
61
42
60
41
38
57
40
39
58
59

FIG 2

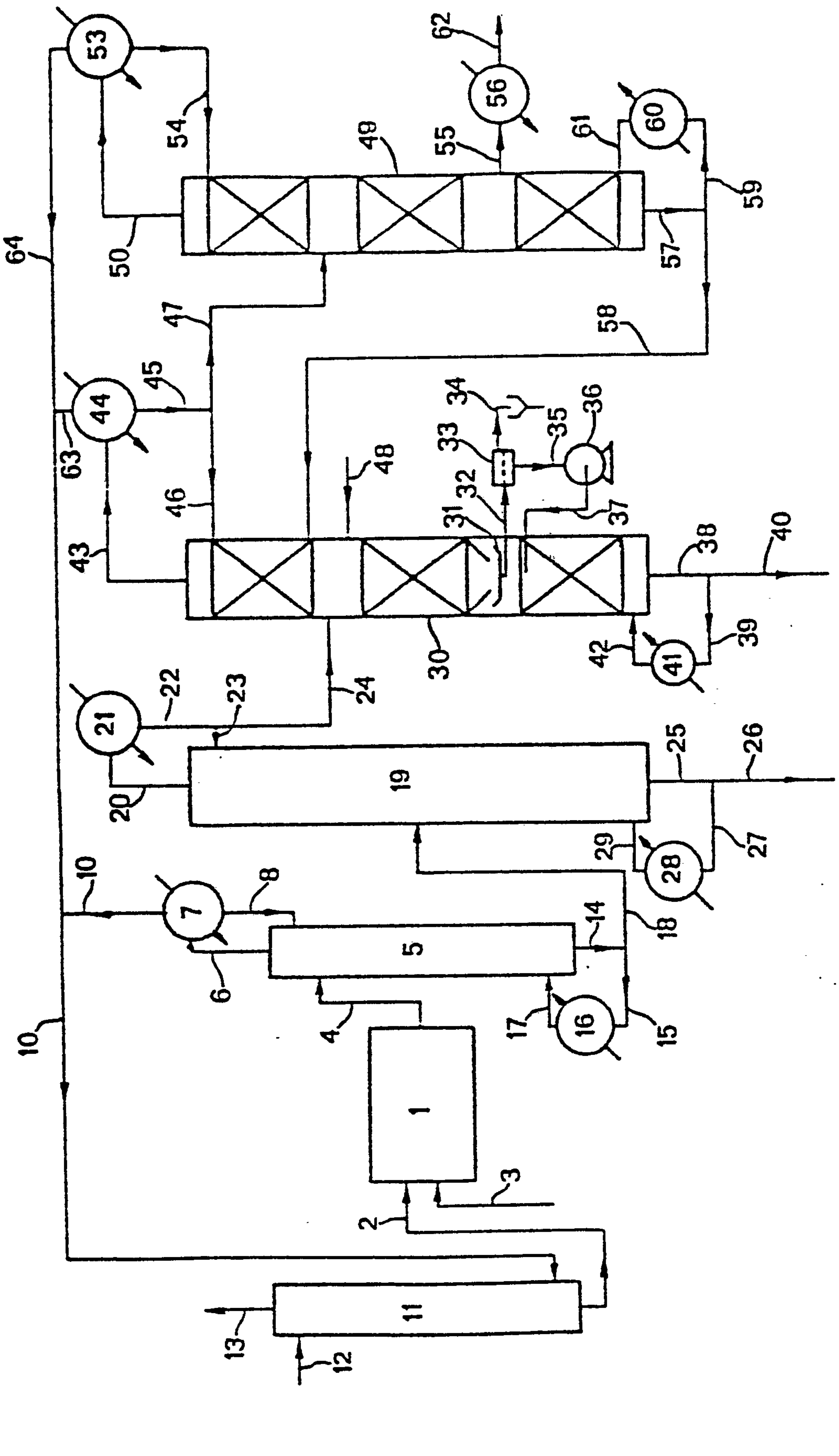
1
2
3
4
5
6
7
8
10
11
12
13
14
15
16
17
18
19
20
21
22
23
24
25
26
27
28
29
30
31
32
33
34
35
36
37
38
39
40
41
42
43
44
45
46
47
48
49
50
53
54
55
56
57
58
59
60
61
62
63
64